# EUROPÄISCHE PATENTSCHRIFT

(11) **EP 1 521 769 B1**
(45) Veröffentlichungstag und Bekanntmachung des Hinweises auf die Patenterteilung: **09.09.2015**
(21) Anmeldenummer: 03762673.6
(22) Anmeldetag: 09.07.2003
(51) Int. Cl.: C07K 5/06, A61K 47/48

(54) **TUBULYSINKONJUGATE**
TUBULYSIN CONJUGATES
CONJUGUES DE TUBULYSINE

(30) Priorität: 09.07.2002 DE 10230875; 11.02.2003 DE 10305531
(43) Veröffentlichungstag der Anmeldung: 13.04.2005
(73) Patentinhaber: Dömling, Alexander, 80797 München (DE)
(72) Erfinder: DOEMLING, Alexander, 80797 München (DE); WEBER, Lutz, 82110 Germering (DE)
(74) Vertreter: Forstmeyer, Dietmar
(86) Internationale Anmeldenummer: PCT/EP2003/007415
(87) Internationale Veröffentlichungsnummer: WO 2004/005326

(56) Entgegenhaltungen:
- WO-A-98/13375
- SASSE F ET AL: "Tubulysins, new cytostatic peptides from myxobacteria acting on microtubuli production, isolation, physico-chemical and biological properties" JOURNAL OF ANTIBIOTICS, JAPAN ANTIBIOTICS RESEARCH ASSOCIATION. TOKYO, JP, Bd. 53, Nr. 9, September 2000 (2000-09), Seiten 879-885, XP009014740 ISSN: 0021-8820
- GREENWALD R B: "PEG drugs: an overview" JOURNAL OF CONTROLLED RELEASE, ELSEVIER SCIENCE PUBLISHERS B.V. AMSTERDAM, NL, Bd. 74, Nr. 1-3, 6. Juli 2001 (2001-07-06), Seiten 159-171, XP004297521 ISSN: 0168-3659
- DUNCAN R ET AL: "Polymer-drug conjugates, PDEPT and PELT: basic principles for design and transfer from the laboratory to clinic" JOURNAL OF CONTROLLED RELEASE, ELSEVIER SCIENCE PUBLISHERS B.V. AMSTERDAM, NL, Bd. 74, Nr. 1-3, 6. Juli 2001 (2001-07-06), Seiten 135-146, XP004297519 ISSN: 0168-3659
- GARNETT MARTIN C: "Targeted drug conjugates: Principles and progress" ADVANCED DRUG DELIVERY REVIEWS, Bd. 53, Nr. 2, 17. Dezember 2001 (2001-12-17), Seiten 171-216, XP002261805 ISSN: 0169-409X
- poster handout
- MOLINEUX G: "Pegylation: engineering improved pharmaceuticals for enhanced therapy", CANCER TREATMENT REVIEWS, vol. 28, no. SupplA, 1 April 2002 (2002-04-01), pages 13-16,

## Beschreibung

Die vorliegende Erfindung betrifft neue Tubulysin-konjugate sowie deren Verwendung zur Behandlung von Krebserkrankungen.

Die Tubulysine wurden erstmals von der Gruppe von Höfle und Reichenbach (GBF Braunschweig) aus einer Kulturbrühe von Stämmen des Myxobakteriums *Archangium gephyra* isoliert (F. Sasse et al. J. Antibiot. 2000, 53, 879-885; WO9813375; DE 10008089). Diese Verbindungen haben eine ausgesprochen hohe cytotoxische Aktivität gegenüber Säugetierzellinien mit IC₅₀-Werten im picomolaren Bereich und sind daher als potentielle Krebsmedikamente von grossem Interesse. 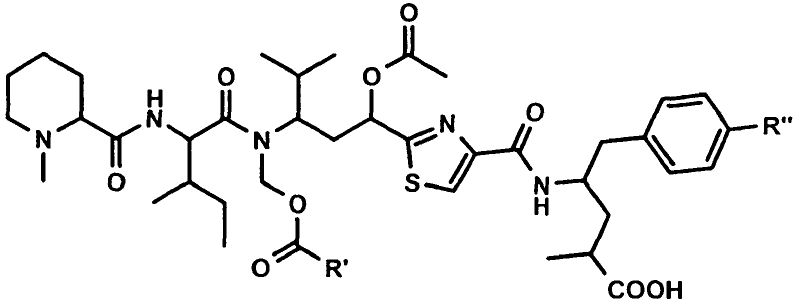
Tubulysin A: R' = CH₂CH (CH₃) ₂; R' ' - OH
Tubulysin B: R' ₌ CH₂CH₂CH₃; R'' = OH
Tubulysin C: R' ₌ CH₂CH₃; R'' = OH
Tubulysin D: R' = CH₂CH(CH₃)₂; R'' - H
Tubulysin E: R' ₌ CH₂CH₂CH₃ ; R'' = H
Tubulysin F: R' ₌ CH₂CH₃; R'' = H

Die extrem hohe Cytotoxizität einiger Tubulysine bringt aber auch Nachteile mit sich: eine hohe allgemeine Toxizität sowie eine geringe Selektivität gegenüber normalen Zellen. Ziel der vorliegenden Erfindung war es daher insbesondere, die Toxizität von Tubulysinen zu senken sowie ihre Selektivität zu steigern.

Diese Aufgabe wird durch Bereitstellung von Tubulysinkonjugaten der allgemeinen Formel U-V-W gelöst, in der
U Tubulysin A ist;
V ein Sauerstoffatom, eine NH-Gruppe oder eine Gruppe der Formel -O-(CR^{a}R^{b})ₙ-O-, -NH-R^{c}-NH-CO-CH₂-O-, -O-R^{c}-O-CO-CH₂-O-oder -O-R^{c}-O- ist, wobei R^{a} und R^{b} unabhängig voneinander C₁-C₆ Alkylgruppen oder zusammen Teil einer Cycloalkylgruppe sind, n gleich 1 oder 2 ist und R^{c} eine Alkylen, Arylen oder Cycloalkylengruppe ist; und
W ein Polyethylenglykol (PEG) mit einem Molekulargewicht von mehr als 30 kDa bis 100 kDa ist.

Erfindungsgemäß wurde also gefunden, dass Polymerkonjugate von speziellen Tubulysinen bessere pharmakologische Eigenschaften und vor allem eine höhere Selektivität bei gegebener Cytotoxizität sowie bei geringerer Toxizität als die unkonjugierten Verbindungen aufweisen. Dies führt dazu, dass im menschlichen und tierischen Körper bevorzugt Krebszellen angesteuert und gesundes Gewebe geschont wird.

Der Ausdruck Alkyl oder Alk bezieht sich auf eine gesättigte, geradkettige oder verzweigte Kohlenwasserstoffgruppe, die 1 bis 20 Kohlenstoffatome, vorzugsweise 1 bis 12 Kohlenstoffatome, besonders bevorzugt 1 bis 6 Kohlenstoffatome aufweist, z.B. die Methyl-, Ethyl-, Propyl-, Isopropyl-, Isobutyl-, tert-Butyl, n-Hexyl-, 2,2-Dimethylbutyl- oder n-Octyl-Gruppe.

Die Ausdrücke Alkenyl und Alkinyl beziehen sich auf zumindest teilweise ungesättigte, geradkettige oder verzweigte Kohlenwasserstoffgruppen, die 2 bis 20 Kohlenstoffatome, vorzugsweise 2 bis 12 Kohlenstoffatome, besonders bevorzugt 2 bis 6 Kohlenstoffatome aufweisen, z. B. die Ethenyl-, Allyl-, Acetylenyl-, Propargyl-, Isoprenyl-oder Hex-2-enyl-Gruppe. Bevorzugt weisen Alkenylgruppen eine oder zwei (besonders bevorzugt eine) Doppelbindungen bzw. Alkinylgruppen eine oder zwei (besonders bevorzugt eine) Dreifachbindungen auf.

Des weiteren beziehen sich die Begriffe Alkyl, Alkenyl und Alkinyl auf Gruppen, bei der ein oder mehrere Wasserstoffatome durch ein Halogenatom (bevorzugt F oder Cl) ersetzt sind wie z. B. die 2,2,2-Trichlorethyl-, oder die Trifluormethylgruppe.

Der Ausdruck Heteroalkyl bezieht sich auf eine Alkyl-, eine Alkenyl- oder eine Alkinyl-Gruppe, in der ein oder mehrere (bevorzugt 1, 2 oder 3) Kohlenstoffatome durch ein Sauerstoff-, Stickstoff-, Phosphor-, Bor-, Selen-, Silizium- oder Schwefelatom ersetzt sind (bevorzugt Sauerstoff, Schwefel oder Stickstoff). Der Ausdruck Heteroalkyl bezieht sich des weiteren auf eine Carbonsäuregruppe oder eine von einer Carbonsäure abgeleitete Gruppe wie z. B. Acyl (Alkyl-CO-), Acylalkyl, Alkoxycarbonyl, Acyloxy, Acyloxyalkyl, Carboxyalkylamid oder Alkoxycarbonyloxy.

Beispiele für Heteroalkylgruppen sind Gruppen der Formeln R^{a}-O-Y^{a}-, R^{a}-S-Y^{a}-, R^{a}-N(R^{b})-Y^{a}-, R^{a}-CO-Y^{a}-, R^{a}-O-CO-Y^{a}-, R^{a}-CO-O-Y^{a}-, R^{a}-CO-N (R^{b}) -Y^{a}-, R^{a}-N (R^{b}) -CO-Y^{a}-, R^{a}-O-CO-N(R^{b}) -Y^{a}-, R^{a}-N(R^{b}) -CO-O-Y^{a}-, R^{a}-N(R^{b}) -CO-N(R^{c}) -Y^{a}-, R^{a}-O-CO-O-Y^{a}-, R^{a}-N(R^{b})-C(=NR^{d})*-*N(R^{c})-Y^{a}-, R^{a}-CS-Y^{a}-, R^{a}-O-CS-Y^{a}-,R^{a}-CS-O-Y^{a}-, R^{a}-CS-N(R^{b})-Y^{a}-, R^{a}-N(R^{b})-CS-Y^{a}-, R^{a}-O-CS-N (R^{b}) -Y^{a}-, R^{a}-N (R^{b}) -CS -O- Y^{a}- , R^{a}-N(R^{b})-CS-N(R^{c})**Y^{a}**-, R^{a}-O-CS-O-Y^{a}-, R^{a}-S-CO-Y^{a}-, R^{a}-CO-S-Y^{a}-, R^{a}-S-CO-N(R^{b})-Y^{a}-, R^{a}-N(R^{b})-CO-S-Y^{a}-, R^{a}**-**S**-**CO**-O-**Y^{a}**-**, R^{a}-O-CO-S-Y^{a}-, R^{a}-S-CO-S-Y^{a}-, R^{a}-S-CS-Y^{a}-, R^{a}-CS-S-Y^{a}-, R^{a}-S-CS-N(R^{b})-Y^{a}-, R^{a}-N(R^{b})-CS-S-Y^{a}-, R^{a}-S-CS-O-Y^{a}-, R^{a}-O-CS-S-Y^{a}-, wobei R^{a} ein Wasserstoffatom, eine C₁-C₆-Alkyl-, eine C₂-C₆-Alkenyl- oder eine C₂-C₆-Alkinylgruppe ; R^{b} ein Wasserstoffatom, eine C₁-C₆-Alkyl-, eine C₂-C₆-Alkenyl- oder eine C₂-C₆- Alkinylgruppe; R^{c} ein Wasserstoffatom, eine C₁-C₆-Alkyl-, eine C₂-C₆-Alkenyl- oder eine C₂-C₆-Alkinylgruppe; R^{d} ein Wasserstoffatom, eine C₁-C₆-Alkyl-, eine C₂-C₆-Alkenyl- oder eine C₂-C₆-Alkinylgruppe und Y^{a} eine direkte Bindung, eine C₁-C₆-Alkylen-, eine C₂-C₆-Alkenylen- oder eine C₂-C₆-Alkinylengruppe ist, wobei jede Heteroalkylgruppe mindestens ein Kohlenstoffatom enthält und ein oder mehrere Wasserstoffatome durch Fluor- oder Chloratome ersetzt sein können. Konkrete Beispiele für Heteroalkylgruppen sind Methoxy, Trifluormethoxy, Ethoxy, n-Propyloxy, iso-Propyloxy, tert-Butyloxy, Methoxymethyl, Ethoxymethyl, Methoxyethyl, Methylamino, Ethylamino, Dimethylamino, Diethylamino, iso-Propylethylamino, Methylaminomethyl, Ethylaminomethyl, Di-iso-Propylaminoethyl, Enolether, Dimethylaminomethyl, Dimethylaminoethyl, Acetyl, Propionyl, Butyryloxy, Acetyloxy, Methoxycarbonyl, Ethoxycarbonyl, N-Ethyl-N-Methylcarbamoyl oder N-Methylcarbamoyl. Weitere Beispiele für Heteroalkylgruppen sind Nitril-, Isonitril, Cyanat-, Thiocyanat-, Isocyanat-, Isothiocyanat und Alkylnitril-gruppen.

Der Ausdruck Cycloalkyl bezieht sich auf eine gesättigte oder teilweise ungesättigte (z. B. Cycloalkenyl) cyclische Gruppe, die einen oder mehrere Ringe (bevorzugt 1 oder 2) aufweist, welche insgesamt 3 bis 14 Ring-Kohlenstoffatome, vorzugsweise 3 bis 10 (insbesondere 3, 4, 5, 6 oder 7) Ring-Kohlenstoffatome enthalten. Der Ausdruck Cycloalkyl bezieht sich weiterhin auf derartige Gruppen, bei denen ein oder mehrere Wasserstoffatome durch Fluor-, Chlor-, Brom- oder Jodatome oder OH, =O, SH, =S, NH₂, =NH oder NO₂-Gruppen ersetzt sind also z. B. cyclische Ketone wie z. B. Cyclohexanon, 2-Cyclohexenon oder Cyclopentanon. Weitere konkrete Beispiele für Cycloalkylgruppen sind die Cyclopropyl-, Cyclobutyl-, Cyclopentyl-, Spiro[4,5]decanyl-, Norborny-, Cyclohexyl-, Cyclopentenyl-, Cyclohexadienyl-, Decalinyl-, Cubanyl-, Bicyclo[4.3.0]nonyl-, Tetralin-, Cyclopentylcyclohexyl-, Fluorcyclohexyl- oder die Cyclohex-2-enyl-Gruppe.

Der Ausdruck Heterocycloalkyl bezieht sich auf eine Cycloalkylgruppe wie oben definiert, in der ein oder mehrere (bevorzugt 1, 2 oder 3) Ring-Kohlenstoffatome durch ein Sauerstoff-, Stickstoff-, Silizium-, Selen-, Phosphor- oder Schwefelatom (bevorzugt Sauerstoff, Schwefel oder Stickstoff) ersetzt sind. Bevorzugt besitzt eine Heterocycloalkylgruppe 1 oder 2 Ringe mit 3 bis 10 (insbesondere 3, 4, 5, 6 oder 7) Ringatomen. Der Ausdruck Heterocycloalkyl bezieht sich weiterhin auf derartige Gruppen, bei denen ein oder mehrere Wasserstoffatome durch Fluor-, Chlor-, Brom- oder Jodatome oder OH, =0, SH, *=_{S},* NH₂, =NH oder NO₂-Gruppen ersetzt sind. Beispiele sind die Piperidyl-, Morpholinyl-, Urotropinyl-, Pyrrolidinyl-, Tetrahydrothiophenyl-, Tetrahydropyranyl-, Tetrahydrofuryl-, Oxacyclopropyl-, Azacyclopropyl- oder 2-Pyrazolinyl-Gruppe sowie Lactame, Lactone, cyclische Imide und cyclische Anhydride.

Der Ausdruck Alkylcycloalkyl bezieht sich auf Gruppen, die entsprechend den obigen Definitionen sowohl Cycloalkyl- wie auch Alkyl-, Alkenyl- oder Alkinylgruppen enthalten, z. B. Alkylcycloalkyl-, Alkylcycloalkenyl-, Alkenylcycloalkyl- und Alkinylcycloalkylgruppen. Bevorzugt enthält eine Alkylcycloalkylgruppe eine Cycloalkylgruppe, die einen oder zwei Ringsysteme aufweist, welche insgesamt 3 bis 10 (insbesondere 3, 4, 5, 6 oder 7) Kohlenstoffatome enthält und eine oder zwei Alkyl-, Alkenyl- oder Alkinylgruppen mit 1 oder 2 bis 6 Kohlenstoffatomen.

Der Ausdruck Heteroalkylcycloalkyl bezieht sich auf Alkylcycloalkylgruppen, wie oben definiert, in der ein oder mehrere (bevorzugt 1, 2 oder 3) Kohlenstoffatome durch ein Sauerstoff-, Stickstoff-, Silizium-, Selen-, Phosphor- oder Schwefelatom (bevorzugt Sauerstoff, Schwefel oder Stickstoff) ersetzt sind. Bevorzugt besitzt eine Heteroalkylcycloalkylgruppe 1 oder 2 Ringsysteme mit 3 bis 10 (insbesondere 3, 4, 5, 6 oder 7) Ringatomen und eine oder zwei Alkyl, Alkenyl, Alkinyl oder Heteroalkylgruppen mit 1 oder 2 bis 6 Kohlenstoffatomen. Beispiele derartiger Gruppen sind Alkylheterocycloalkyl, Alkylheterocycloalkenyl, Alkenylheterocycloalkyl, Alkinylheterocycloalkyl, Heteroalkylcycloalkyl, Heteroalkylheterocycloalkyl und Heteroalkylheterocylcloalkenyl, wobei die cyclischen Gruppen gesättigt oder einfach, zweifach oder dreifach ungesättigt sind.

Der Ausdruck Aryl bzw. Ar bezieht sich auf eine aromatische Gruppe, die einen oder mehrere Ringe hat, die insgesamt 6 bis 14 Ring-Kohlenstoffatome, vorzugsweise 6 bis 10 (insbesondere 6) Ring-Kohlenstoffatome enthalten. Der Ausdruck Aryl (bzw. Ar) bezieht sich weiterhin auf derartige Gruppen, bei denen ein oder mehrere Wasserstoffatome durch Fluor-, Chlor-, Brom- oder Jodatome oder OH, SH, NH₂, oder NO₂ - Gruppen ersetzt sind. Beispiele sind die Phenyl-, Naphthyl-, Biphenyl-, 2-Fluorphenyl, Anilinyl-, 3-Nitrophenyl oder 4-Hydroxyphenyl-Gruppe.

Der Ausdruck Heteroaryl bezieht sich auf eine aromatische Gruppe, die einen oder mehrere Ringe hat, welche insgesamt das 5 bis 14 Ringatome, vorzugsweise 5 bis 10 (insbesondere 5 oder 6) Ringatome enthalten und ein oder mehrere (bevorzugt 1, 2, 3 oder 4) Sauerstoff-, Stickstoff-, Phosphor- oder Schwefel-Ringatome (bevorzugt O, S oder N) enthalten. Der Ausdruck Heteroaryl bezieht sich weiterhin auf derartige Gruppen, bei denen ein oder mehrere Wasserstoffatome durch Fluor-, Chlor-, Brom- oder Jodatome oder OH, SH, NH₂, oder NO₂-Gruppen ersetzt sind. Beispiele sind 4-Pyridyl-, 2-Imidazolyl-, 3-Phenylpyrrolyl-, Thiazolyl-, Oxazolyl-, Triazolyl-, Tetrazolyl-, Isoxazolyl-, Indazolyl-, Indolyl-, Benzimidazolyl-, Pyridazinyl-, Chinolinyl-, Purinyl-, Carbazolyl-, Acridinyl-, Pyrimidyl-, 2,3'-Bifuryl-, 3-Pyrazolyl- und Isochinolinyl-Gruppen.

Der Ausdruck Aralkyl bezieht sich auf Gruppen, die entsprechend den obigen Definitionen sowohl Aryl- wie auch Alkyl-, Alkenyl-, Alkinyl- und/oder Cycloalkylgruppen enthalten, wie z. B. Arylalkyl-, Arylalkenyl-, Arylalkinyl-, Arylcycloalkyl-, Arylcycloalkenyl-, Alkylarylcycloalkyl- und Alkylarylcycloalkenylgruppen. Konkrete Beispiele für Aralkyle sind Toluol, Xylol, Mesitylen, Styrol, Benzylchlorid, O - Fluortoluol, 1H-Inden, Tetralin, Dihydronaphthaline, Indanon, Phenylcyclopentyl, Cumol, Cyclo-hexylphenyl, Fluoren und Indan. Bevorzugt enthält eine Aralkylgruppe ein oder zwei aromatische Ringsysteme (1 oder 2 Ringe) mit insgesamt 6 bis 10 Ring-Kohlenstoffatomen und ein oder zwei Alkyl-, Alkenyl- und/oder Alkinylgruppen mit 1 oder 2 bis 6 Kohlenstoffatomen und/oder eine Cyclo-alkylgruppe mit 5 oder 6 Ringkohlenstoffatomen.

Der Ausdruck Heteroaralkyl bezieht sich auf eine Aralkylgruppe wie oben definiert, in der ein oder mehrere (bevorzugt 1, 2, 3 oder 4) Kohlenstoffatome durch ein Sauerstoff-, Stickstoff-, Silizium-, Selen-, Phosphor-, Boroder Schwefelatom (bevorzugt Sauerstoff, Schwefel oder Stickstoff) ersetzt sind, d. h. auf Gruppen, die entsprechend den obigen Definitionen sowohl Aryl- bzw. Heteroaryl- wie auch Alkyl-, Alkenyl-, Alkinyl- und/oder Heteroalkyl- und/oder Cycloalkyl- und/oder Heterocycloalkylgruppen enthalten. Bevorzugt enthält eine Heteroaralkylgruppe ein oder zwei aromatische Ringsysteme (1 oder 2 Ringe) mit insgesamt 5 oder 6 bis 10 Ring-Kohlenstoffatomen und ein oder zwei Alkyl-, Alkenyl- und/oder Alkinylgruppen mit 1 oder 2 bis 6 Kohlenstoffatomen und/oder eine Cycloalkylgruppe mit 5 oder 6 Ringkohlenstoffatomen, wobei 1, 2, 3 oder 4 dieser Kohlenstoffatome durch Sauerstoff-, Schwefel- oder Stickstoffatome ersetzt sind.

Beispiele sind Arylheteroalkyl-, Arylheterocycloalkyl-, Arylheterocycloalkenyl-, Arylalkylheterocycloalkyl-, Arylalkenylheterocycloalkyl-, Arylalkinylheterocyclo-alkyl-, Arylalkylheterocycloalkenyl-, Heteroarylalkyl-, Heteroarylalkenyl-, Heteroarylalkinyl-, Heteroarylheteroalkyl-, Heteroarylcycloalkyl-, Heteroarylcycloalkenyl-, Heteroarylheterocycloalkyl-, Heteroarylheterocycloalkenyl-, Heteroarylalkylcycloalkyl-, Heteroarylalkylheterocycloalkenyl-, Heteroarylheteroalkylcycloalkyl-, Heteroarylheteroalkylcycloalkenyl- und Heteroarylheteroalkylheterocycloalkyl-Gruppen, wobei die cyclischen Gruppen gesättigt oder einfach, zweifach oder dreifach ungesättigt sind. Konkrete Beispiele sind die Tetrahydroisochinolinyl-, Benzoyl-, 2- oder 3-Ethylindolyl-, 4-Methylpyridino-, 2-, 3- oder 4-Methoxyphenyl-, 4-Ethoxyphenyl-, 2-, 3- oder 4-Carboxyphenylalkylgruppe.

Die Ausdrücke Cycloalkyl, Heterocycloalkyl, Alkylcycloalkyl, Heteroalkylcycloalkyl, Aryl, Heteroaryl, Aralkyl und Heteroaralkyl beziehen sich auch auf Gruppen, in denen ein oder mehrere Wasserstoffatome solcher Gruppen durch Fluor-, Chlor-, Brom- oder Jodatome oder OH, =O, SH, =S, NH₂, =NH oder NO₂ - Gruppen ersetzt sind.

Der Ausdruck "gegebenenfalls substituiert" bezieht sich auf Gruppen, in denen ein oder mehrere Wasserstoffatome durch Fluor-, Chlor-, Brom- oder Jodatome oder OH, =0, SH, =S, NH₂, =NH oder NO₂ - Gruppen ersetzt sind. Dieser Ausdruck bezieht sich weiterhin auf Gruppen, die ausschließlich oder zusätzlich mit unsubstituierten C₁-C₆ Alkyl-, C₂-C₆ Alkenyl-, C₂-C₆ Alkinyl-, C₁-C₆ Heteroalkyl-, C₃-C₁₀ Cycloalkyl-, C₂-C₉ Heterocycloalkyl-, C₆-C₁₀ Aryl-, C₁-C₉ Heteroaryl-, C₇-C₁₂ Aralkyl- oder C₂-C₁₁ Heteroaralkyl-Gruppen substituiert sind.

Die beschriebenen Verbindungen können aufgrund ihrer Substitution ein oder mehrere Chiralitätszentren enthalten. Die vorliegende Erfindung umfasst daher sowohl alle reinen Enantiomere und alle reinen Diastereomere, als auch deren Gemische in jedem Mischungsverhältnis.

Besonders bevorzugt weisen die erfindungsgemässen Konjugate (U-V-W) die folgende Formel auf, wobei die Stereochemie der des natürlichen Tubulysin A entspricht:

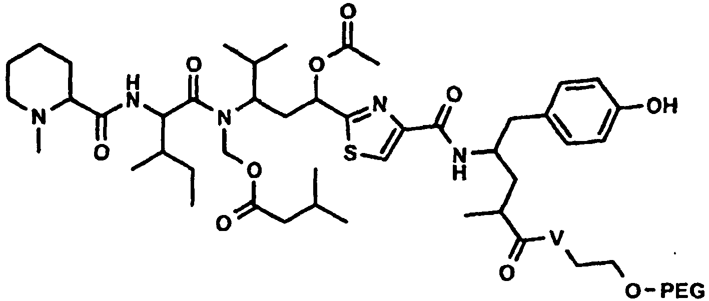

Pharmakologisch akzeptable Salze, Solvate, Hydrate oder Formulierungen der hier beschriebenen Konjugate sind ebenfalls Gegenstand der vorliegenden Erfindung. Beispiele für pharmakologisch akzeptable Salze der beschriebener Verbindungen Salze von physiologisch akzeptablen Mineralsäuren wie Salzsäure, Schwefelsäure und Phosphorsäure oder Salze von organischen Säuren wie Methansulfonsäure, p-Toluolsulfonsäure, Milchsäure, Essigsäure, Ameisensäure, Trifluoressigsäure, Zitronensäure, Bernsteinsäure, Fumarsäure, Maleinsäure und Salicylsäure. Verbindungen können solvatisiert, insbesondere hydratisiert sein. Die Hydratisierung kann z.B. während des Herstellungsverfahrens oder als Folge der hygroskopischen Natur der anfänglich wasserfreien Verbindungen auftreten.

Die pharmazeutischen Zusammensetzungen gemäß der vorliegenden Erfindung enthalten mindestens eine Verbindung gemäß Anspruch 1 als Wirkstoff und fakultativ Trägerstoffe und/oder Adjuvantien.

Die therapeutische Verwendung der Verbindungen gemäß Anspruch 1, ihrer pharmakologisch akzeptablen Salze bzw. Solvate und Hydrate sowie Formulierungen und pharmazeutischen Zusammensetzungen liegt ebenfalls im Rahmen der vorliegenden Erfindung.

Auch die Verwendung dieser Wirkstoffe zur Herstellung von Arzneimitteln zur Behandlung von Krebserkrankungen ist Gegenstand der vorliegenden Erfindung. Des weiteren sind die vorliegenden Verbindungen bei der Vorbeugung und/oder Behandlung von Pilzerkrankungen (d.h. als antifugale Mittel), rheumatoider Arthritis, entzündlichen Erkrankungen, Immunologisch bedingten Krankheiten (z. B. Diabetes Typ 1), Autoimmunkrankheiten sowie weiteren Tumorerkrankungen von großem Interesse. Im allgemeinen werden Verbindungen gemäß Anspruch 1 unter Anwendung der bekannten und akzeptablen Modi, entweder einzeln oder in Kombination mit einem beliebigen anderen therapeutischen Mittel verabreicht. Solche therapeutisch nützlichen Mittel werden bevorzugt parenteral, z.B. als injizierbare Lösung verabreicht. Zur Herstellung von flüssigen Lösungen kann man Arzneimittelträgerstoffe wie z.B. Wasser, Alkohole oder wäßrige Salzlösungen verwenden. Die pharmazeutisch verwendbaren Mittel können auch Zusatzstoffe zur Konservierung, Stabilisierung, Salze zur Veränderung des osmotischen Drucks, Puffer und Antioxidantien enthalten.

Kombinationen mit anderen therapeutischen Mitteln können weitere Wirkstoffe beinhalten, die gewöhnlich zur Behandlung von Krebserkrankungen eingesetzt werden.

### Beispiele

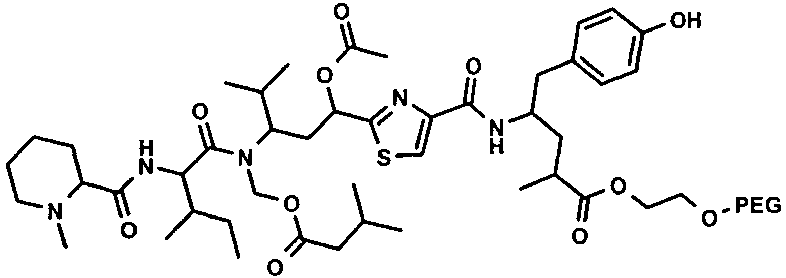

Zu einer Lösung von 0.056 mmol Tubulysin A und 0.125 mmol PEG (6 kDa, 10 kDa, 20 kDa, 35 kDa bzw. 40 kDa) in einem Gemisch aus 3 ml Acetonitril und 1 ml DMF wurden bei 0°C 0.1 mmol 2-Chloro-1-methylpyridiniumiodid (Mukaiyama Reagenz) und 0.2 mmol 4-Dimethylaminopyridin (DMAP) zugegeben. Die Reaktionsmischung wurde 2h bei 0°C und weitere 60h bei Raumtemperatur gerührt. Anschliessend wurde die Mischung einrotiert, mit 15 ml Dichlormethan versetzt und mit je 5 ml Wasser, NaHCO_{3(aq)}, Wasser sowie gesättigter Kochsalzlösung gewaschen. Die organische Phase wurde über Na₂SO₄ getrocknet und das Lösungsmittel im Vakuum entfernt. Der Rückstand wurde in 8 ml DCM gelöst, unter Rühren mit trockenem Ether versetzt, bis die Lösung trüb wurde und für eine Stunde stehengelassen. Der ausgefallene Feststoff wurde abfiltriert und mit Ether gewaschen. Die gewünschten Produkte wurden als weisses Pulver erhalten.

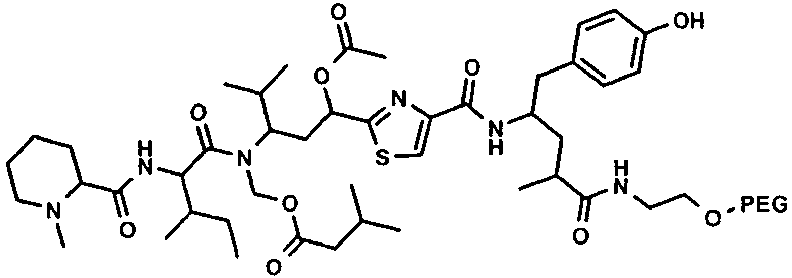

Die Diamine der Polyethylenglycole (6 kDa, 10 kDa, 20 kDa, 35 kDa bzw. 40 kDa) sowie ihre Verknüpfung mit Tubulysin A wurden analog zu dem in R. B. Greenwald et al. Bioorg. Med. Chem. 1998, 6, 551-562 beschriebenen Verfahren hergestellt.

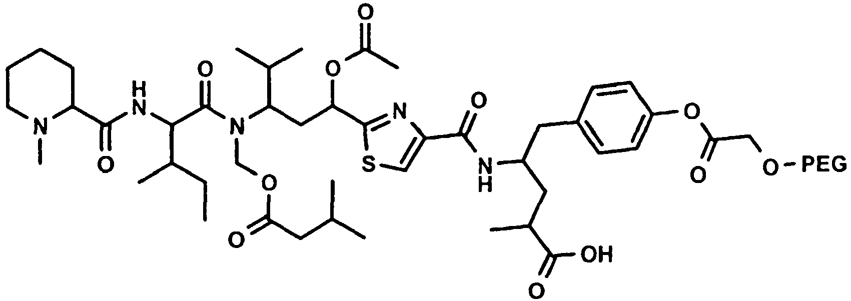

Die PEG Dicarbonsäuren (6 kDa, 10 kDa, 20 kDa, 35 kDa bzw. 40 kDa) sowie ihre Verknüpfung mit Tubulysin A wurden analog zu dem in R. B. Greenwald et al. J. Med. Chem. 1996, 39, 424-431 beschriebenen Verfahren hergestellt.

## Patentansprüche

1. Verbindungen der allgemeinen Formel U-V-W, wobei
U Tubulysin A ist;
V ein Sauerstoffatom, eine NH-Gruppe oder eine Gruppe der Formel -O- (CR^{a}R^{b})ₙ-O-, -NH-R^{c}-NH-CO-CH₂ -O- , -O-R^{c}-O-CO-CH₂-O- oder -O-R^{c}-O- ist, wobei R^{a} und R^{b} unabhängig voneinander C₁-C₆ Alkylgruppen oder zusammen Teil einer Cycloalkylgruppe sind, n gleich 1 oder 2 ist und R^{c} eine Alkylen, Arylen oder Cycloalkylengruppe ist; und
W ein Polyethylenglykol (PEG) mit einem Molekulargewicht von mehr als 30 kDa bis 100 kDa ist.

2. Verbindungen nach Anspruch 1, die folgende Formel aufweisen, wobei die Stereochemie der des natürlichen Tubulysin A entspricht: 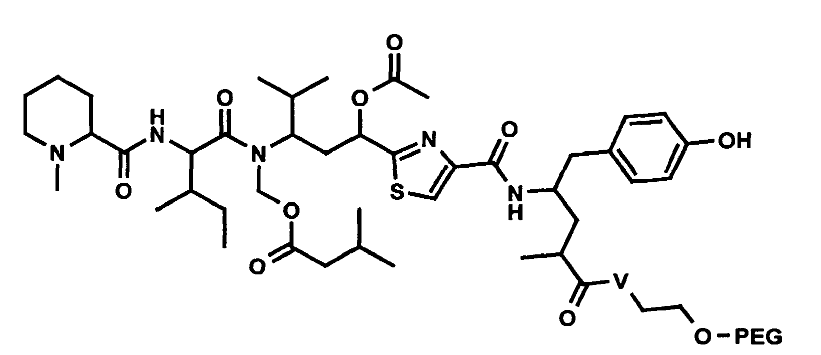 wobei V wie in Anspruch 1 definiert ist und PEG ein Polyethylenglykol mit einem Molekulargewicht von mehr als 30 kDa bis 100 kDa ist.

3. Verbindungen nach Anspruch 1 oder 2 zur Verwendung zur Behandlung von Krebserkrankungen.

## Claims

1. Compounds of general formula U-V-W, wherein
U is Tubulysin A;
V is an oxygen atom, a NH group or a group of the formula -O-(CR^{a}R^{b})ₙ-O-, -NH-R^{c}-NH-CO-CH₂-O-, -O-R^{c}-O-CO- CH₂-O- or -O-R^{c}-O-, wherein R^{a} and R^{b} independently from each other are C₁-C₆ alkyl groups or together are part of a cycloalkyl group, n is equal to 1 or 2 and R^{c} is an alkylene, arylene or a cycloalkylene group; and
W is a polyethylene glycol (PEG) having a molecular weight of more than 30 kDa to 100 kDa.

2. Compounds according to claim 1 having the following formula wherein the stereochemistry corresponds to that of natural Tubulysin A: 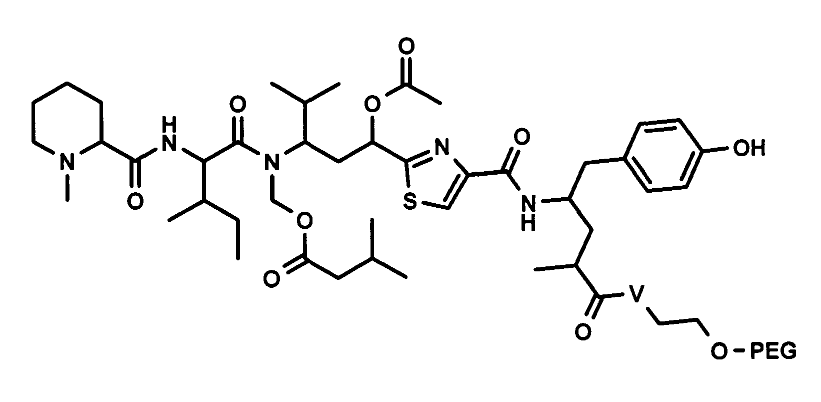 wherein V is defined as in claim 1 and PEG is a polyethylene glycol having a molecular weight of more than 30 kDa to 100 kDa.

3. Compounds according to claim 1 or 2 for use for the treatment of cancer diseases.

## Revendications

1. Composés de la formule générale U-V-W, dans laquelle
U est la tubulysine A ;
V est un atome d'oxygène, un groupe NH- ou un groupe de formule -O-(CR^{a}R^{b})ₙ-O-, -NH-R^{c}-NH-CO-CH₂-O-, -O-R^{c}-O-CO-CH₂-O-ou -O-R^{c}-O-, où R^{a} et R^{b} sont indépendamment l'un de l'autre des groupes alkyles en C1-C₆ ou constituent ensemble une partie d'un groupe cycloalkyle, n est égal à 1 ou 2 et R^{c} est un groupe alkylène, arylène ou cycloalkylène ; et
W est un polyéthylène glycol (PEG) avec une masse moléculaire supérieure à une valeur entre 30 kDa et 100 kDa.

2. Composés selon la revendication 1, qui présentent la formule suivante, dans laquelle la stéréochimie correspond à celle de la tubulysine A naturelle : 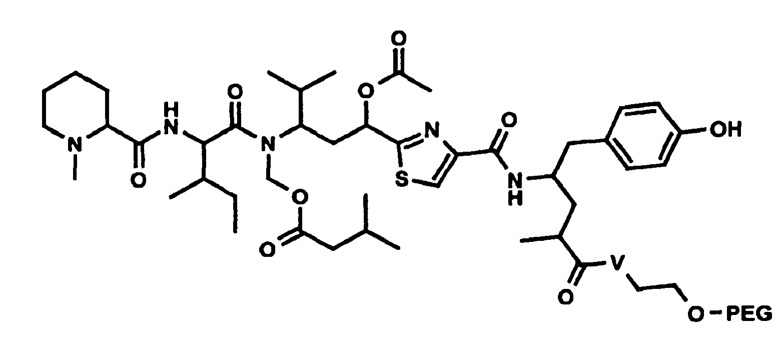 dans laquelle V est tel que défini dans la revendication 1 et PEG est un polyéthylène glycol avec une masse moléculaire supérieure à une valeur entre 30 kDa et 100 kDa.

3. Composés selon les revendications 1 ou 2, destinés à une utilisation pour le traitement de maladies cancéreuses.
